# EUROPÄISCHE PATENTSCHRIFT

(11) **EP 0 500 639 B1**
(45) Veröffentlichungstag und Bekanntmachung des Hinweises auf die Patenterteilung: **03.08.1994**
(21) Anmeldenummer: 90916479.0
(22) Anmeldetag: 03.11.1990
(51) Int. Cl.: C07C 315/04, C07C 317/14

(54) **VERFAHREN ZUR HERSTELLUNG VON ALKYL-[3-CHLORPHENYL]-SULFONEN**
PROCESS FOR THE PRODUCTION OF ALKYL-(3-CHLOROPHENYL) SULPHONES
PROCEDE DE FABRICATION DE SULFONES D'ALKYLE-3-CHLOROPHENYLE

(30) Priorität: 09.11.1989 DE 3937282
(43) Veröffentlichungstag der Anmeldung: 02.09.1992
(73) Patentinhaber: HOECHST AKTIENGESELLSCHAFT, 65926 Frankfurt am Main (DE)
(72) Erfinder: SELL, Günther, D-6242 Kronberg (DE); SEMLER, Günther, D-6233 Kelkheim (DE)
(86) Internationale Anmeldenummer: EP9001846
(87) Internationale Veröffentlichungsnummer: WO9107384

(56) Entgegenhaltungen:
- EP-A- 0 187 285
- DE-A- 2 160 148
- DE-C- 949 054
- US-A- 4 675 447
- CHEMICAL ABSTRACTS, Band 62, Nr. 6, 1965, Columbus, Ohio, US; H. KUGITA et al: "Diuretics. I. Alkylsulfonyltoluene derivatives", Zusammenfassung 6422c-6424d (6422e)

## Beschreibung

Die Erfindung betrifft ein verfahren zur Herstellung von Alkyl-[3-chlorphenyl]-sulfonen durch Umsetzung von Alkylphenylsulfonen mit Chlor in Schwefelsäure oder Oleum.

Es ist bekannt, daß beispielsweise Methyl-[4-methylphenyl]-sulfon durch Umsetzung mit Chlor in Gegenwart von Antimon(III)-chlorid ohne Lösungsmittel bei 85 bis 90°C in Methyl-[3-chlor-4-methyl]-phenylsulfon überführt werden kann (H. Kugita et al., Chem. Pharm. Bull. 10, 1001-8, CA 62 6422c). Das nach diesem Verfahren gewonnene Produkt ist jedoch durch höherchlorierte Nebenprodukte verunreinigt (US 46 75 447, S.1, Sp.25). Ferner wurde die Chlorierung von Methyl-[4-methylphenyl]-sulfon mit Sulfurylchlorid in Gegenwart von Metallchloriden, wie Eisen(III)-chlorid oder Antimon(III)-chlorid bei 90 bis 92°C durchgeführt (US 46 75 447). Die dort beschriebenen Verfahrensergebnisse konnten beim Nacharbeiten nicht bestätigt werden. Hinzu kommt, daß bei diesem Verfahren große Mengen an Katalysator benötigt werden.

Eine weitere Möglichkeit zur Herstellung von Alkylarylsulfonen besteht in der Oxidation der entsprechenden Thioether (DE-OS 21 60 148), bei deren Herstellung jedoch Isomerenprobleme auftreten (D. Chianelli et al. Synthesis 1982 475-8).

Es bestand somit ein Bedürfnis für ein besseres Verfahren zur Herstellung von Alkyl-[3-chlorphenyl]-sulfonen, bei welchem die Nachteile der bekannten Verfahren vermieden werden.

Es wurde nun gefunden, daß man Alkyl-[3-chlorphenyl]-sulfone der allgemeinen Formel (I) 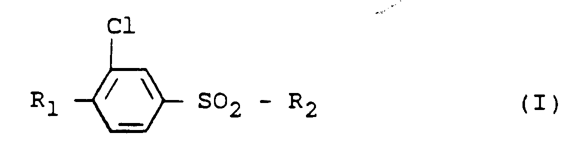
in welcher R₁ und R₂ geradkettige oder verzweigte Alkyl(C₁-C₄)-Gruppen, die gleich oder verschieden sein können, bedeuten,vorteilhaft herstellen kann, indem man Alkylphenylsulfone der allgemeinen Formel (II) 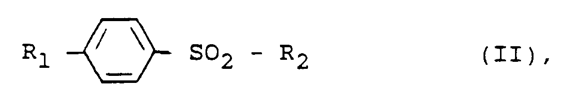
in welcher R₁ und R₂ die vorstehend genannten Bedeutungen haben, in Schwefelsäure oder Oleum mit Chlor bei Temperaturen von etwa 20 bis etwa 180°C, zweckmäßigerweise von etwa 50 bis etwa 120°C, vorzugsweise von etwa 60 bis etwa 90°C, umsetzt.

Die als Reaktionsmedium dienende Schwefelsäure kann in einer Konzentration von etwa 50 bis 100 %, oder auch in Form von bis zu 65 %igem Oleum eingesetzt werden. Vorzugsweise verwendet man jedoch eine Schwefelsäure von etwa 70 bis etwa 96 %. Beim Arbeiten in Schwefelsäure von Konzentratonen unter 70 % kann es zur Ausfällung des Produktes schon während der Umsetzung und dadurch bedingte Einschlüsse von Ausgangsprodukt kommen. Oleum ist aus wirtschaftlichen Gründen und sicherheitstechnischen Überlegungen weniger geeignet und bietet auch hinsichtlich Ausbeute und erzielter Produktreinheit keinerlei Vorteile.

Die Menge der eingesetzten Schwefelsäure ist unkritisch. Bei Anwendung von mehr als einem Gewichtsteil Ausgangsprodukt je Gewichtsteil Schwefelsäure kann es jedoch zu Rührproblemen bei der Isolierung des Produktes kommen. Bei der Verwendung sehr großer Schwefelsäuremengen besteht die Möglichkeit von Ausbeuteverlusten infolge unvollständiger Isolierung des Produkts während der Aufarbeitung. Zur optimalen Reaktionsführung setzt man üblicherweise 1 bis 3, vorzugsweise 2 Gewichtsteile Schwefelsäure je Gewichtsteil Ausgangsprodukt ein.

Die Chlorierung nach dem erfindungsgemäßen Verfahren ist bei Temperaturen von etwa 20 bis etwa 180°C möglich. Bei Temperaturen unter 50°C ist die Reaktionsgeschwindigkeit gering, während oberhalb 120°C mehr höherchlorierte und verfärbte Produkte entstehen. Zweckmäßigerweise wird ein Temperaturbereich von etwa 50 bis etwa 120°C, vorzugsweise von etwa 60 bis etwa 90°C angewandt.

Ferner kann die Chlorierung sowohl bei Normaldruck als auch bei Überdruck, vorzugsweise jedoch bei Normaldruck, durchgeführt werden.

Es wird solange Chlor eingeleitet, bis das Ausgangsprodukt möglichst weitgehend umgesetzt ist. Aus der so gewonnenen Reaktionsmischung wird das Produkt durch Zugabe von Wasser ausgefällt bzw. die bereits zum Teil vorhandene Fällung vervollständigt. Anschließend wird das Produkt durch Filtration isoliert.

Eine ökologisch vorteilhafte Verfahrensdurchführung besteht darin, nach der Reaktion die Schwefelsäure durch Zugabe von Wasser bis auf etwa 70 % zu verdünnen und das auskristallisierte Reaktionsprodukt aus der Reaktionsmischung zu isolieren. Als Mutterlauge fällt eine leicht regenerierbare, salzfreie Abfallschwefelsäure an. Man erhält auf diesem Wege die erfindungsgemäßen Produkte in hoher Ausbeute und Reinheit.

Ein weiterer Vorteil des vorliegenden Verfahrens besteht darin, daß die Chlorierung ohne Verwendung der üblichen Kernchlorierungs-Katalysatoren abläuft. Sie kann selbstverständlich auch in Gegenwart von Halogenierungs-Katalysatoren, wie Eisen(III)-chlorid oder Antimon(III)-chlorid, durchgeführt werden.

Außer durch Fällung kann das Produkt auch durch Extraktion, beispielsweise mit Xylol oder halogenierten Kohlenwasserstoffen, aus der Mutterlauge gewonnen werden. Es wird aus dem Extrakt durch Kristallisation oder Destillation isoliert.

Alkyl-[3-chlorphenyl]-sulfone, insbesondere Methyl-[3-chlor-4-methylphenyl]-sulfon, sind wichtige Zwischenprodukte zur Herstellung von Pflanzenschutzmitteln (EP 268 795).

### Beispiel 1

In einen 500 ml Glaskolben mit Rührer, Kühler, Gaseinleitung, Thermometer und Tropftrichter werden 85,1 g (0,5 mol) Methyl-[4-methylphenyl)-sulfon in 160 g 96 %iger Schwefelsäure vorgelegt. Bei 80°C dosiert man innerhalb von 6 Stunden unter gutem Rühren ca. 75 g Chlor zu. Nach beendeter Reaktion entfernt man restlichen Chlorwasserstoff mittels Durchleiten eines N₂-Stroms, tropft langsam 58 g Wasser zu und läßt dann unter Rühren innerhalb von 2 Stunden bis auf 20°C abkühlen. Man filtriert das ausgefallene Produkt ab und wäscht mit ca. 500 ml Wasser bis zur Säurefreiheit. Nach dem Trocknen bei 50°C/100 mbar erhält man 96,5 g 3-Chlor-4-methylphenyl-methylsulfon (Gehalt 98,6 Gew.-%, entsprechend einer Ausbeute von 93,0 % d.Th., Schmp.: 91,5°C).

### Beispiel 2

In einen 500 ml Glaskolben mit Rührer, Kühler, Gaseinleitung, Thermometer und Tropftrichter werden 92,1 g (0,5 mol) Ethyl-[4-methylphenyl]-sulfon mit 160 g 96 %iger Schwefelsäure vorgelegt. Bei 70°C dosiert man innerhalb von 6 Stunden unter gutem Rühren ca. 210 g Chlor zu. Nach beendeter Reaktion entfernt man restlichen Chlorwasserstoff mittels Durchleiten eines N₂-Stroms, tropft langsam 40 g Wasser zu und läßt dann unter Rühren innerhalb von 2 Stunden bis auf 20°C abkühlen. Man filtriert das ausgefallene Produkt ab und wäscht mit ca. 130 ml Wasser bis zur Säurefreiheit. Nach dem Trocknen bei 30°C/100 mbar erhält man 100,7 g 3-Chlor-4-methylphenyl-ethylsulfon (Gehalt 98,5 Gew.-%, entsprechend einer Ausbeute von 90,7 % d.Th., Schmp.: 54°C).

### Beispiel 3

In einem 500 ml Glaskolben mit Rührer, Kühler, Gaseinleitung, Thermometer und Tropftrichter werden 99,1 g (0,5 mol) Isopropyl-[4-methylphenyl]-sulfon in 180 g 96 %iger Schwefelsäure vorgelegt. Bei 70°C dosiert man innerhalb von 6 Stunden unter gutem Rühren ca. 140 g Chlor zu. Nach beendeter Reaktion entfernt man restlichen Chlorwasserstoff mittels Durchleiten eines N₂-Stroms, tropft 300 g Wasser zu und läßt bis auf 20°C abkühlen. Man extrahiert viermal mit je 200 g Methylenchlorid, wäscht die vereinten Extrakte bis zur Neutralität mit 5%iger NaHCO₃-Lösung, trocknet mit Na₂SO₄ und engt am Rotationsverdampfer ein. Man erhält 66,7 g 3-Chlor-4-methylphenyl-isopropylsulfon (gelbliches Öl, Gehalt 94,3 Gew.-%, entsprechend einer Ausbeute von 54,1 %, ¹H-NMR (CDCl₃): 7,3-7,9 (m, 3H); 3,23 (sp, 6,8 Hz, 1H); 2,47 (s); 1,42 d, 6,8 Hz, 6H).

### Beispiel 4

In einem 500 ml Glaskolben mit Rührer, Kühler, Gaseinleitung, Thermometer und Tropftrichter werden 85,1 g (0,5 mol) Methyl-[4-methylphenyl)-sulfon in 150 g 10,6 %igem Oleum vorgelegt.
Bei 65°C dosiert man innerhalb von 5 Stunden unter gutem Rühren ca. 120 g Chlor zu. Nach beendeter Reaktion entfernt man restlichen Chlorwasserstoff mittels Durchleiten eines N₂-Stroms, tropft 69 g Wasser zu und läßt bis auf 20°C abkühlen. Man filtriert das ausgefallene Produkt ab und wäscht mit ca. 500 ml Wasser bis zur Säurefreiheit. Nach dem Trocknen bei 50°C/100 mbar erhält man 65,4 g 3-Chlor-4-methylphenyl-methylsulfon (Gehalt 98,5 Gew.-%, entsprechend einer Ausbeute von 62,9 % d.Th., Schmp.: 89-91°C).

## Patentansprüche

1. Verfahren zur Herstellung von Alkyl-[3-chlorphenyl]-sulfonen der allgemeinen Formel (I) 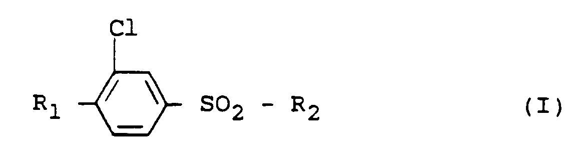 in welcher R₁ und R₂ geradkettige oder verzweigte Alkyl(C₁-C₄)-Gruppen, die gleich oder verschieden sein können, bedeuten, dadurch gekennzeichnet, daß man Alkyl-phenylsulfone der allgemeinen Formel (II) 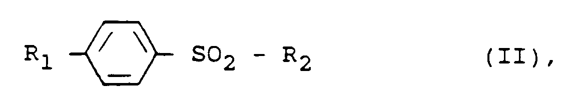 in welcher R₁ und R₂ die vorstehend genannten Bedeutungen haben, in Schwefelsäure oder Oleum mit Chlor bei Temperaturen von etwa 20 bis etwa 180°C umsetzt.

2. Verfahren nach Anspruch 1, dadurch gekennzeichnet, daß man bei Temperaturen von etwa 50 bis etwa 120°C umsetzt.

3. Verfahren nach mindestens einem der Ansprüche 1 und 2, dadurch gekennzeichnet, daß man bei Temperaturen von etwa 60 bis etwa 90°C umsetzt.

4. Verfahren nach mindestens einem der Ansprüche 1 bis 3, dadurch gekennzeichnet, daß man in etwa 50 bis 100%iger Schwefelsäure umsetzt.

5. Verfahren nach mindestens einem der Ansprüche 1 bis 4, dadurch gekennzeichnet, daß man in etwa 70 bis etwa 96 %iger Schwefelsäure umsetzt.

6. Verfahren nach mindestens einem der Ansprüche 1 bis 3, dadurch gekennzeichnet, daß man in bis zu 65 %igem Oleum umsetzt.

7. Verfahren nach mindestens einem der Ansprüche 1 bis 5, dadurch gekennzeichnet, daß man die Umsetzung in 96%iger Schwefelsäure durchführt und die Schwefelsäurekonzentration nach der Umsetzung durch Zugabe von Wasser auf etwa 70 % herabsetzt.

8. Verfahren nach mindestens einem der Ansprüche 1 bis 7, dadurch gekennzeichnet, daß man bei Normal- oder Überdruck umsetzt.

9. Verfahren nach mindestens einem der Ansprüche 1 bis 8, dadurch gekennzeichnet, daß R₁ und R₂ in den allgemeinen Formeln I und II von Anspruch 1 je eine Methylgruppe bedeutet.

## Claims

1. Process for the preparation of alkyl 3-chlorophenyl sulfones of the formula (I) 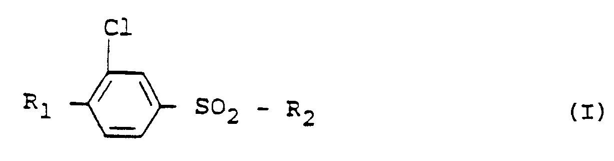 in which R₁ and R₂ are straight-chain or branched alkyl (C₁-C₄) groups, which can be identical or different, characterized in that alkyl phenyl sulfones of the general formula (II) 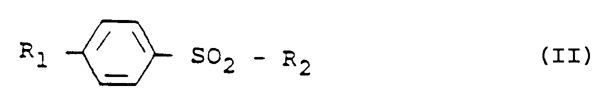 in which R₁ and R₂ have the meanings given above, are reacted with chlorine in sulfuric acid or fuming sulfuric acid at temperatures of about 20 to about 180°C.

2. Process according to Claim 1, characterized in that the reaction is carried out at temperatures of about 50 to about 120°C.

3. Process according to at least one of Claims 1 and 2, characterized in that the reaction is carried out at temperatures of about 60 to about 90°C.

4. Process according to at least one of Claims 1 to 3, characterized in that the reaction is carried out in about 50 to 100 % strength sulfuric acid.

5. Process according to at least one of Claims 1 to 4, characterized in that the reaction is carried out in about 70 to about 96 % strength sulfuric acid.

6. Process according to at least one of Claims 1 to 3, characterized in that the reaction is carried out in up to 65 % strength fuming sulfuric acid.

7. Process according to at least one of Claims 1 to 5, characterized in that the reaction is carried out in 96 % strength sulfuric acid and the sulfuric acid concentration after the reaction is reduced to about 70 % by addition of water .

8. Process according to at least one of Claims 1 to 7, characterized in that atmospheric pressure or super-atmospheric pressure is employed.

9. Process according to at least one of Claims 1 to 8, characterized in that R₁ and R₂ in the formulae I and II of Claim 1 are each a methyl group.

## Revendications

1. Procédé pour produire des alkyl-(3-chlorophényl)-sulfones de formule générale (I) : 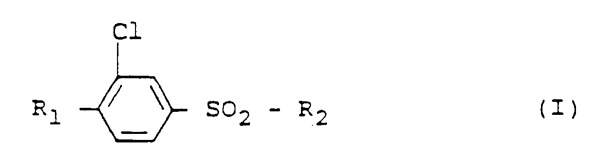 (dans laquelle R₁ et R₂ représentent des groupes alkyles (en C₁ à C₄), linéaires ou ramifiés, qui peuvent être identiques ou différents), procédé caractérisé en ce qu'on fait réagir des alkyl-phényl-sulfones de formule générale (II) : 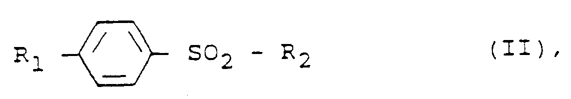 (dans laquelle R₁ et R₂ ont les sens précités) dans de l'acide sulfurique ou dans de l'oléum, avec du chlore à des températures d'environ 20 à environ 180°C.

2. Procédé selon la revendication 1, caractérisé en ce qu'on fait réagir à des températures d'environ 50 à environ 120°C.

3. Procédé selon l'une au moins des revendications 1 et 2, caractérisé en ce qu'on fait réagir à des températures d'environ 60 à environ 90°C;

4. Procédé selon l'une au moins des revendications 1 à 3, caractérisé en ce qu'on fait réagir dans de l'acide sulfurique ayant une concentration d'environ 50 à environ 100°C.

5. Procédé selon l'une au moins des revendications 1 à 4, caractérisé en ce qu'on fait réagir dans de l'acide sulfurique ayant une concentration d'environ 70 à environ 96 %.

6. Procédé selon l'une au moins des revendications 1 à 3, caractérisé en ce qu'on fait réagir dans un milieu comportant jusqu'à 65 % d'oléum.

7. Procédé selon l'une au moins des revendications 1 à 5, caractérisé en ce qu'on conduit la réaction dans de l'acide sulfurique à 96 % et, après la réaction, on diminue jusqu'à 70 % environ la concentration de l'acide sulfurique en lui ajoutant de l'eau.

8. Procédé selon l'une au moins des revendications 1 à 7, caractérisé en ce qu'on fait réagir sous la pression normale ou sous une surpression.

9. Procédé selon l'une au moins des revendications 1 à 8, caractérisé en ce que, dans les formules générales I et II de la revendication 1, R₁et R₂ représentent chacun un groupe méthyle.
